# EUROPÄISCHE PATENTSCHRIFT

(11) **EP 4 198 011 B1**
(45) Veröffentlichungstag und Bekanntmachung des Hinweises auf die Patenterteilung: **31.07.2024**
(21) Anmeldenummer: 21215368.8
(22) Anmeldetag: 17.12.2021
(51) Int. Cl.: C07C 45/50, C07C 47/02

(54) **VERFAHREN ZUR HYDROFORMYLIERUNG VON OLEFINEN UNTER EINSATZ VON PT UND DPEPHOS**
METHOD FOR HYDROFORMYLATION OF OLEFINS USING PT AND DPEPHOS
PROCÉDÉ D'HYDROFORMYLATION D'OLÉFINES À L'AIDE DU PT ET DU DPEPHOS

(43) Veröffentlichungstag der Anmeldung: 21.06.2023
(73) Patentinhaber: Evonik Oxeno GmbH & Co. KG, 45772 Marl (DE)
(72) Erfinder: SCHNEIDER, Carolin, 40789 Monheim am Rhein (DE); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(56) Entgegenhaltungen:
- MEESSEN P ET AL: "Highly regioselective hydroformylation of internal, functionalized olefins applying Pt/Sn complexes with large bite angle diphosphines", JOURNAL OF ORGANOMETALLIC CHEMISTRY, ELSEVIER, AMSTERDAM, NL, vol. 551, no. 1-2, 30 January 1998 (1998-01-30), pages 165 - 170, XP004197061, ISSN: 0022-328X, DOI: 10.1016/S0022-328X(97)00396-3

## Beschreibung

Die vorliegende Erfindung betrifft ein Verfahren zur Hydroformylierung von Olefinen unter Einsatz von Pt und DPEphos.

In P. Meessen et al., Journal of Organometallic Chemistry, 551, (1998), 165-170 wird der Einsatz von DPEphosPtCl₂ zur Hydroformylierung von Methyl-3-pentenoat beschrieben.

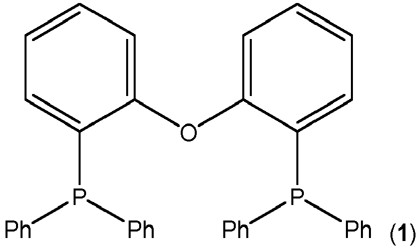

### (DPEphos)

Der vorliegende Erfindung lag die Aufgabe zugrunde, eine neues Hydroformylierungsverfahren bereitzustellen. Das Verfahren soll hierbei eine gegenüber dem aus dem Stand der Technik bekannten Verfahren gesteigerte Ausbeute liefern.

Diese Aufgabe wird gelöst durch ein Verfahren gemäß Anspruch 1.

Verfahren umfassend die Verfahrensschritte:
a) Vorlegen eines Olefins;
b) Zugabe einer Verbindung gemäß der Formel (I): 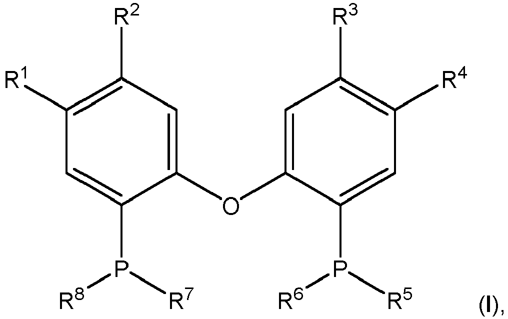 wobei R¹, R², R³, R⁴, R⁵, R⁶, R⁷, R⁸ ausgewählt sind aus: -H, -(C₁-C₁₂)-Alkyl, -(C₆-C₂₀)-Aryl, und mindestens zwei der Reste R⁵, R⁶, R⁷, R⁸ für -(C₆-C₂₀)-Aryl stehen;
c) Zugabe einer Pt-Verbindung, welche zur Ausbildung eines Komplexes befähigt ist;
d) Zugabe einer Brom-Verbindung oder einer lod-Verbindung;
e) Zuführen von CO und H₂;
f) Erwärmen des Reaktionsgemisches aus a) bis e), wobei das Olefin zu einem Aldehyd umgesetzt wird.

Hierbei können die Verfahrensschritte a) bis e) in beliebiger Reihenfolge erfolgen. Üblicherweise erfolgt die Zugabe von CO und H₂ jedoch, nachdem die Reaktionspartner in den Schritten a) bis d) vorgelegt wurden.

Hierbei können die Verfahrensschritte c) und d) in einem Schritt, durch Zugabe von PtBr₂ oder PtI₂, erfolgen.

In einer bevorzugten Variante des Verfahrens erfolgt die Zugabe der Pt-Verbindung und der Brom-Verbindung oder lod-Verbindung in einem Schritt, durch Zugabe von PtBr₂ oder PtI₂.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, *n*-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

In einer Variante des Verfahrens stehen R⁵, R⁶, R⁷, R⁸ für -(C₆-C₂₀)-Aryl.

In einer Variante des Verfahrens stehen R⁵, R⁶, R⁷, R⁸ für -Ph.

In einer Variante des Verfahrens stehen R¹ und R⁴ für -H.

In einer Variante des Verfahrens R² und R³ für -H.

In einer Variante des Verfahrens weist die Verbindung gemäß der Formel (I) die Struktur (1) auf:

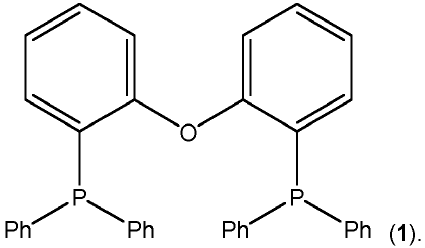

In einer Variante des Verfahrens ist die Pt-Verbindung ausgewählt aus: Pt(II)I₂, Pt(II)Br₂, Pt(IV)I₄, Pt(IV)Br₄, Diphenyl(1,5-COD)Pt(II), Pt(II)(acac)₂, Pt(0)(PPh₃)₄, Pt(0)(DVTS)-Lösung (CAS:68478-92-2), Pt(0)(Ethylen)(PPh₃)₂, Pt(II)Br₂(COD), Tris(benzylidenaceton)Pt(0), Pt(II)(OAC)₂-Lösung, Pt(0)(t-Bu)₂, Pt(II)(COD)Me₂, Pt(II)(COD)I₂, Pt(IV)IMe₃, Pt(II)(Hexafluoroacetylacetonat)₂.

In einer Variante des Verfahrens ist die Pt-Verbindung ausgewählt aus: Pt(II)I₂, Pt(II)Br₂.

In einer Variante des Verfahrens ist die lod-Verbindung beziehungsweise die Brom-Verbindung ausgewählt aus: Alkalihalogenid, Erdalkalihalogenid, NH₄X, Alkylammoniumhalogenid, Dialkylhalogenid, Trialkylhalogenid, Tetraalkylhalogenid, Cycloalkylammoniumhalogenid.

In einer Variante des Verfahrens wird in Verfahrensschritt d) eine Brom-Verbindung zugegeben, die Pt(II)Br₂ ist.

In einer Variante des Verfahrens wird die Brom-Verbindung in einer Menge zugegeben, gemessen in Äquivalenten bezogen auf Pt, welche im Bereich von 0.1 bis 10 liegt.

In einer Variante des Verfahrens wird in Verfahrensschritt d) eine lod-Verbindung zugegeben, die Pt(II)I₂ ist.

In einer Variante des Verfahrens wird die lod-Verbindung in einer Menge zugegeben, gemessen in Äquivalenten bezogen auf Pt, welche im Bereich von 0.1 bis 10 liegt.

In einer Variante des Verfahrens umfasst dieses den zusätzlichen Verfahrensschritt e'): e') Zugabe eines Lösungsmittels.

In einer Variante des Verfahrens ist das Lösungsmittel ausgewählt aus: THF, DCM, ACN, Heptan, DMF, Toluol, Texanol, Pentan, Hexan, Octan, Isooctan, Decan, Dodecan, Cyclohexan, Benzen, Xylene, Marlotherm, Propylencarbonat, MTBE, Diglyme, Triglyme, Diethylether, Dioxan, Isopropanol, Tertbutanol, Isononanol, Isobutanol, Isopentanol, Ethylacetat.

In einer Variante des Verfahrens ist das Lösungsmittel ausgewählt aus: THF, DCM, ACN, Heptan, DMF, Toluol, Texanol.

In einer Variante des Verfahrens erfolgt das Zuführen von CO und H₂ bei einem Druck in einem Bereich von 1 MPa (10 bar) bis 6 MPa (60 bar).

In einer Variante des Verfahrens erfolgt das Zuführen von CO und H₂ bei einem Druck in einem Bereich von 1 MPa (20 bar) bis 6 MPa (50 bar).

In einer Variante des Verfahrens erfolgt das Erwärmen auf eine Temperatur im Bereich von 25 °C bis 150°C.

In einer Variante des Verfahrens erfolgt das Erwärmen auf eine Temperatur im Bereich von 30 °C bis 130°C.

In einer Variante des Verfahrens ist das Olefin ausgewählt aus: Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

Im Folgenden soll die Erfindung anhand eines Ausführungsbeispieles näher erläutert werden.

### Versuchsbeschreibung

Ein Vial wurde mit PtX₂ (X = Halogen), Ligand, und einem im Ofen getrockneten Rührstab bestückt. Dann wird das Vial mit Septum (PTFE-beschichteter StyrolButadien-Kautschuk) und Phenolharzdeckel verschlossen. Das Vial wird dreimal evakuiert und mit Argon wieder befüllt. Mit einer Spritze wurden Toluol und 1-Octen in das Vial gegeben. Das Vial wurde in eine Legierungsplatte gestellt, die in einen Autoklav der Reihe 4560 von Parr Instruments unter Argon Atmosphäre überführt wurde. Nach dreimaligem Spülen des Autoklaven mit CO/H₂ wurde der Synthesegasdruck auf 40 bar bei Raumtemperatur erhöht. Die Reaktion wurde 20 h bei 120 °C durchgeführt. Nach Beendigung der Reaktion wurde der Autoklav auf Raumtemperatur abgekühlt und vorsichtig entspannt. Der Ausbeute und Selektivität wurden durch GC-Analyse bestimmt.

### Hydroformylierung von 1-Octen

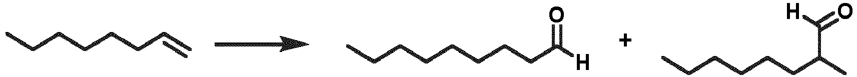

### Reaktionsbedingungen:

10.0 mmol 1-Octen, 0.1 mol% PtX₂, 2.2 Äquivalente Ligand, Lösungsmittel: Toluol, p(CO/H₂): 40 bar, T: 120 °C, t: 20 h.

Ausbeuten:

| Ligand | Halogen | Ausbeute [%] |
|---|---|---|
| 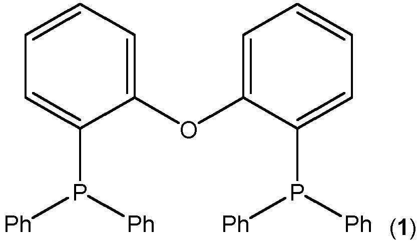 | I / Br / Cl | 76 / 45 / 14 |

Wie die Versuchsergebnisse zeigen, wird die Aufgabe durch das erfindungsgemäße Verfahren gelöst. Der Versuch, in dem das Halogen (X) CI ist, ist ein Vergleichsversuch.

## Patentansprüche

1. Verfahren umfassend die Verfahrensschritte:
a) Vorlegen eines Olefins;
b) Zugabe einer Verbindung gemäß der Formel (I): 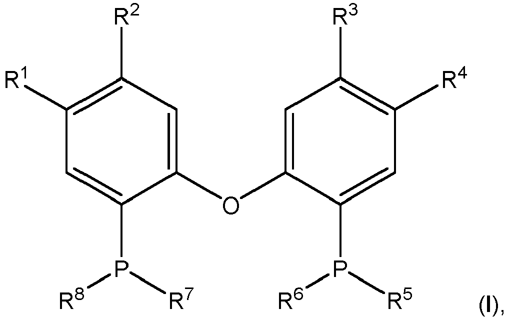 wobei R¹, R², R³, R⁴, R⁵, R⁶, R⁷, R⁸ ausgewählt sind aus: -H, -(C₁-C₁₂)-Alkyl, -(C₆-C₂₀)-Aryl, und mindestens zwei der Reste R⁵, R⁶, R⁷, R⁸ für -(C₆-C₂₀)-Aryl stehen;
c) Zugabe einer Pt-Verbindung, welche zur Ausbildung eines Komplexes befähigt ist;
d) Zugabe einer Brom-Verbindung oder einer lod-Verbindung;
e) Zuführen von CO und H₂;
f) Erwärmen des Reaktionsgemisches aus a) bis e), wobei das Olefin zu einem Aldehyd umgesetzt wird.

2. Verfahren nach Anspruch 1,
wobei R⁵, R⁶, R⁷, R⁸ für -(C₆-C₂₀)-Aryl stehen.

3. Verfahren nach einem der Ansprüche 1 oder 2,
wobei R⁵, R⁶, R⁷, R⁸ für -Ph stehen.

4. Verfahren nach einem der Ansprüche 1 bis 3,
wobei R¹ und R⁴ für -H stehen.

5. Verfahren nach einem der Ansprüche 1 bis 4,
wobei R² und R³ für -H stehen.

6. Verfahren nach einem der Ansprüche 1 bis 5,
wobei die Verbindung gemäß der Formel (I) die Struktur (1) aufweist:

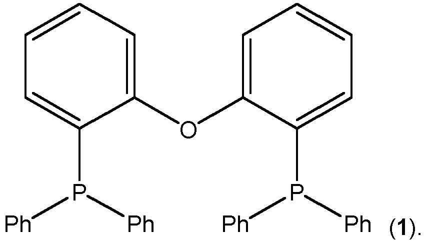

7. Verfahren nach einem der Ansprüche 1 bis 6,
wobei die Pt-Verbindung ausgewählt ist aus: Pt(II)I₂, Pt(II)Br₂, Pt(IV)I₄, Pt(IV)Br₄, Diphenyl(1,5-COD)Pt(II), Pt(II)(acac)₂, Pt(0)(PPh₃)₄, Pt(0)(DVTS)-Lösung (CAS:68478-92-2), Pt(0)(Ethylen)(PPh₃)₂, Pt(II)Br₂(COD), Tris(benzylidenaceton)Pt(0), Pt(II)(OAC)₂-Lösung, Pt(0)(t-Bu)₂, Pt(II)(COD)Me₂, Pt(II)(COD)I₂, Pt(IV)IMe₃, Pt(II)(Hexafluoroacetylacetonat)₂.

8. Verfahren nach einem der Ansprüche 1 bis 7,
wobei in Verfahrensschritt d) eine Brom-Verbindung zugegeben wird, die Pt(II)Br₂ ist.

9. Verfahren nach Anspruch 8,
wobei die Brom-Verbindung in einer Menge zugegeben wird, gemessen in Äquivalenten bezogen auf Pt, welche im Bereich von 0.1 bis 10 liegt.

10. Verfahren nach einem der Ansprüche 1 bis 7,
wobei in Verfahrensschritt d) eine lod-Verbindung zugegeben wird, die Pt(II)I₂ ist.

11. Verfahren nach Anspruch 10,
wobei die lod-Verbindung in einer Menge zugegeben wird, gemessen in Äquivalenten bezogen auf Pt, welche im Bereich von 0.1 bis 10 liegt.

12. Verfahren nach einem der Ansprüche 1 bis 11,
umfassend den zusätzlichen Verfahrensschritt e'):
e') Zugabe eines Lösungsmittels.

13. Verfahren nach Anspruch 12,
wobei das Lösungsmittel ausgewählt ist aus: THF, DCM, ACN, Heptan, DMF, Toluol, Texanol, Pentan, Hexan, Octan, Isooctan, Decan, Dodecan, Cyclohexan, Benzen, Xylene, Marlotherm, Propylencarbonat, MTBE, Diglyme, Triglyme, Diethylether, Dioxan, Isopropanol, Tertbutanol, Isononanol, Isobutanol, Isopentanol, Ethylacetat.

14. Verfahren nach einem der Ansprüche 1 bis 13,
wobei das Zuführen von CO und H₂ bei einem Druck erfolgt in einem Bereich von 1 MPa (10 bar) bis 6 MPa (60 bar).

15. Verfahren nach einem der Ansprüche 1 bis 14,
wobei das Erwärmen auf eine Temperatur erfolgt im Bereich von 25 °C bis 150°C.

## Claims

1. Process comprising the process steps of:
a) initially charging an olefin;
b) adding a compound of formula (I): 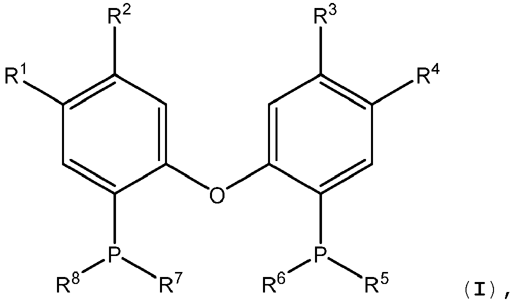 where R¹, R², R³, R⁴, R⁵, R⁶, R⁷, R⁸ are selected from: -H, -(C₁-C₁₂)-alkyl, -(C₆-C₂₀)-aryl, and at least two of the R⁵, R⁶, R⁷, R⁸ radicals are -(C₆-C₂₀)-aryl;
c) adding a Pt compound capable of forming a complex;
d) adding a bromine compound or an iodine compound;
e) feeding in CO and H₂;
f) heating the reaction mixture from steps a) to e), to convert the olefin to an aldehyde.

2. Process according to Claim 1,
where R⁵, R⁶, R⁷, R⁸ are -(C₆-C₂₀)-aryl.

3. Process according to either of Claims 1 and 2,
where R⁵, R⁶, R⁷, R⁸ are -Ph.

4. Process according to any of Claims 1 to 3,
where R¹ and R⁴ are -H.

5. Process according to any of Claims 1 to 4,
where R² and R³ are -H.

6. Process according to any of Claims 1 to 5,
wherein the compound of formula (I) has the structure (1) :

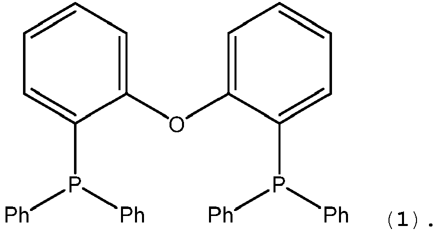

7. Process according to any of Claims 1 to 6,
wherein the Pt compound is selected from: Pt(II)I₂, Pt(II)Br₂, Pt(IV)I₄, Pt(IV)Br₄, diphenyl(1,5-COD)Pt(II), Pt(II)(acac)₂, Pt(0)(PPh₃)₄, Pt(0)(DVTS) solution (CAS:68478-92-2), Pt(0) (ethylene)(PPh₃)₂, Pt(II)Br₂(COD), tris(benzylideneacetone)Pt(0), Pt(II) (OAC) 2 solution, Pt(0)(t-Bu)₂, Pt(II)(COD)Me₂, Pt(II)(COD)I₂, Pt(IV)IMe₃, Pt(II)(hexafluoroacetylacetonate)₂.

8. Process according to any of Claims 1 to 7,
wherein a bromine compound which is Pt(II)Br₂ is added in process step d).

9. Process according to Claim 8,
wherein the bromine compound is added in an amount in the range of 0.1 to 10, measured in equivalents based on Pt.

10. Process according to any of Claims 1 to 7,
wherein in process step d) an iodine compound is added, which is Pt(II)I₂.

11. Process according to Claim 10,
wherein the iodine compound is added in an amount in the range of 0.1 to 10, measured in equivalents based on Pt.

12. Process according to any of Claims 1 to 11,
comprising the additional process step e'):
e') adding a solvent.

13. Process according to Claim 12,
wherein the solvent is selected from: THF, DCM, ACN, heptane, DMF, toluene, texanol, pentane, hexane, octane, isooctane, decane, dodecane, cyclohexane, benzene, xylene, Marlotherm, propylene carbonate, MTBE, diglyme, triglyme, diethyl ether, dioxane, isopropanol, tert-butanol, isononanol, isobutanol, isopentanol, ethyl acetate.

14. Process according to any of Claims 1 to 13,
wherein CO and H₂ are fed in at a pressure in a range from 1 MPa (10 bar) to 6 MPa (60 bar).

15. Process according to any of Claims 1 to 14,
wherein the reaction mixture is heated to a temperature in the range from 25°C to 150°C.

## Revendications

1. Procédé comprenant les étapes suivantes :
a) mise en place d'une oléfine ;
b) addition d'un composé de Formule (I) : 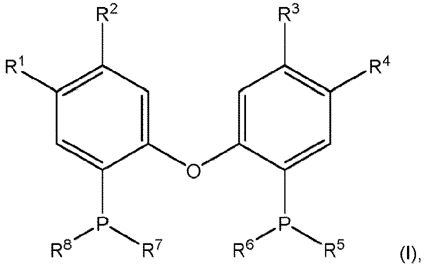 dans laquelle R¹, R², R³, R⁴, R⁵, R⁶, R⁷, R⁸ sont choisis parmi -H, un -alkyle en C₁-C₁₂, un -aryle en C₆-C₂₀, et au moins deux des radicaux R⁵, R⁶, R⁷, R⁸ représentent un -aryle en C₆-C₂₀,
c) addition d'un composé de Pt qui permet la formation d'un complexe ;
d) addition d'un composé du brome ou d'un composé de l'iode ;
e) amenée de CO et de H₂ ;
f) chauffage du mélange réactionnel de a) à e), l'oléfine étant convertie en un aldéhyde.

2. Procédé selon la revendication 1, dans lequel R⁵, R⁶, R⁷, R⁸ représentent un -aryle en C₆-C₂₀.

3. Procédé selon l'une des revendications 1 ou 2, dans lequel R⁵, R⁶, R⁷, R⁸ représentent -Ph.

4. Procédé selon l'une des revendications 1 à 3, dans lequel R¹ et R⁴ représentent -H.

5. Procédé selon l'une des revendications 1 à 4, dans lequel R² et R³ représentent -H.

6. Procédé selon l'une des revendications 1 à 5, dans lequel le composé de formule (I) présente la structure (1) :

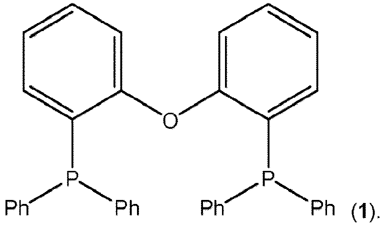

7. Procédé selon l'une des revendications 1 à 6, dans lequel le composé de Pt est choisi parmi Pt(II)I₂, Pt(II)Br₂, Pt(IV)I₄, Pt(IV)Br₄, diphényl (1,5-COD)Pt(II), Pt(II)(acac)₂, Pt(0)(PPh₃)₄, une solution de Pt(0)(DVTS) (CAS: 68478-92-2), Pt(0) (éthylène) (PPh₃)₂, Pt(II)Br₂(COD), tris(benzylidèneacétone)Pt(0), solution de Pt(II)(OAC)₂, Pt(0)(t-Bu)₂, Pt(II)(COD)Me₂, Pt(II)(COD)I₂, Pt(IV)IMe₃, Pt(II)(hexafluoroacétylacétonate)₂.

8. Procédé selon l'une des revendications 1 à 7, dans lequel, dans l'étape d), on ajoute un composé du brome, qui est Pt(II)Br₂.

9. Procédé selon la revendication 8, dans lequel le composé du brome est ajouté en une quantité, exprimée en équivalents par rapport à Pt, comprise dans la plage de 0,1 à 10.

10. Procédé selon l'une des revendications 1 à 7, dans lequel, dans l'étape d), on ajoute un composé de l'iode, qui est Pt(II)I₂.

11. Procédé selon la revendication 10, dans lequel le composé de l'iode est ajouté en une quantité, exprimée en équivalents par rapport à Pt, comprise dans la plage de 0,1 à 10.

12. Procédé selon l'une des revendications 1 à 11, comprenant l'étape supplémentaire e') : e') addition d'un solvant.

13. Procédé selon la revendication 12, dans lequel le solvant est choisi parmi les composés THF, DCM, ACN, heptane, DMF, toluène, texanol, pentane, hexane, octane, isooctane, décane, dodécane, cyclohexane, benzène, xylène, Marlotherm, carbonate de propylène, MTBE, diglyme, triglyme, diéthyléther, dioxane, isopropanol, tert-butanol, isononanol, isobutanol, isopentanol, acétate d'éthyle.

14. Procédé selon l'une des revendications 1 à 13, dans lequel l'amenée du CO et du H₂ a lieu sous une pression dans la plage de 1 MPa (10 bar) à 6 MPa (60 bar).

15. Procédé selon l'une des revendications 1 à 14, dans lequel le chauffage a lieu à une température de 25 °C à 150 °C.
